# EUROPEAN PATENT APPLICATION

(11) **EP 2 837 379 A1**
(43) Date of publication of application: **18.02.2015**
(21) Application number: 14180600.0
(22) Date of filing: 12.08.2014
(51) Int. Cl.: A61K 9/48, A61K 9/16, A61K 9/50, A61K 31/18, A61K 31/473, A61K 45/06

(54) **Dosage form comprising a 5-alpha-reductase steroid inhibitor and an alpha blocker, process for the preparation of a dosage form, use of dosage form, method for the treatment or prophylaxis of a disease or condition mediated by androgen**

(30) Priority: 12.08.2013 BR 102013020508
(71) Applicant: EMS S.A., 13186-901 Sao Paulo - SP (BR)
(72) Inventor: Santus, Giancarlos, MILANO (IT); Soldati, Giuseppe, BOLLATE (IT); Donadoni, Luca, ALZANO LOMBARDO (IT)
(74) Representative: Cabinet Plasseraud

(57) **Abstract**

The present invention describes new and improved dosage forms containing a steroid 5-alpha-reductase inhibitor, in combination with an alpha blocker, for the treatment or prophylaxis of an androgen mediated disease or condition, preferably for the treatment of benign prostatic hyperplasia (HPB).

## Description

### Field of the Invention

This invention relates to improved dosage forms of a steroid 5-alpha-reductase inhibitor, in combination with an alpha blocker, for the treatment or prophylaxis of a disease or condition mediated by androgen, preferably for the treatment or prophylaxis of benign prostatic hyperplasia (BPH). In particular, the present invention relates to pharmaceutical compositions of tamsulosin and dutasteride with good bioavailability, better patient compliance, and a method for obtaining such improved dosage forms containing tamsulosin and dutasteride.

### Background of the Invention

Globally, benign prostatic hyperplasia has affected about 210 million men since 2010 (6% of the population). The prostate becomes larger in most men as they age. For a 46-year-old man, free of symptoms, the risk of developing BPH during the next 30 years is 45%. Incidence rates increase from three cases per 1,000 man-years at age 45-49, to 38 cases per 1,000 man-years by the age of 75-79. While the prevalence rate is 2.7% for men aged 45-49, it increases to 24% by the age of 80 years. (Verhamme KM et al. Eur Urol. Oct 2002, 42 (4): 323-8).

Benign prostatic hyperplasia symptoms are classified as storage or voiding. Storage symptoms include urinary frequency, urgency (compelling need to urinate that cannot be delayed), urgency incontinence, and voiding at night (nocturia). Urinary symptoms include hesitation of urinary flow (need to wait for the stream to begin), intermittency (when the stream starts and stops intermittently) and straining to void. These storage and voiding symptoms are evaluated using the International Prostate Symptom Score questionnaire (International Prostate Symptom Score-IPSS) to assess the severity of BPH.

The two main drugs in the management of BPH are alpha-blockers and 5-alpha-reductase inhibitors.

5-alpha-reductase inhibitors interfere with the effect of certain androgens (male hormones) in the prostate. This slows the growth of the prostate and can even decrease its size, which may help to improve the symptoms of benign prostatic hyperplasia (BPH). 5-alpha-reductase inhibitors (5-ARIs) are also used to treat male pattern baldness.

Drugs in this class are finasteride (marketed as Proscar®) or Propecia®) and dutasteride (marketed as Avodart®). The difference between finasteride and dutasteride is that dutasteride inhibits the activities of both types of the enzyme 5AR1 and 5AR2, unlike finasteride, which only inhibits type 2. These enzymes are responsible for the conversion of testosterone to DHT (dihydrotestosterone). DHT is the main cause of prostate growth and hair loss and dutasteride reduces DHT levels by 90 percent, after only two weeks.

Dutasteride works by reducing the size of the prostate, which helps to relieve symptoms of BPH such as difficulty starting the flow of urine, weak stream, and the need to urinate frequently or urgently (including during the middle of the night). This can also reduce the need for surgery to treat BPH.

Tamsulosin hydrochloride (marketed as Flomax®, Flomaxtra®, Urimax®) is a selective alpha-1-blocker used in the symptomatic treatment of benign prostatic hyperplasia. Tamsulosin is used in men to treat the symptoms of prostatic hyperplasia (BPH) by relaxing the muscles in the prostate and bladder, which helps to improve urine flow and reduces the symptoms of BPH.

Other alpha blockers are alfuzosin, doxazosin, phentolamine, phenoxybenzamine, prazosin, terazosin, silodosin, etc.

The synthesis, pharmacological activities and formulations of 5-alpha-reductase inhibitors and alpha-blockers are described in various patents, including, for example, US 4377584, US 4703063, US 4772475, US 5565467 and WO2010092596, incorporated by reference herein.

Combinations of tamsulosin and dutasteride are also known and reported, for example, in WO2006099121 which describes depot injectable formulations in the form of nanoparticles, in DE 10218611, which describes the combination of two active ingredients without specific examples of dosage forms or formulation, and in WO2006055659, which describes the combination of dutasteride and tamsulosin in several formulations in hard capsule form, containing modified-release pellets of tamsulosin and a soft gelatin capsule of dutasteride. Each of these patents is incorporated by reference herein. In particular, WO20006055659 describes a fixed dose combination in the form of a capsule of hydroxypropylmethylcellulose containing dutasteride dissolved in a soft gelatin capsule and tamsulosin in the form of multi-coated beads with modifying release polymers. In the present application, dutasteride is present in granulated form as a solid dispersion, with a hydrophilic polymer contained together with tamsulosin in various coated beads with modified release polymers in a hard gelatin capsule.

### Summary of the Invention

An embodiment of the present invention is a combination of a steroid 5 alpha-reductase inhibitor and an alpha blocker, in the formulation containing the steroid 5-alpha-reductase inhibitor as a solid dispersion and the alpha blocker in the form of a modified release formulation.

A first embodiment of the invention relates to an improved dosage form containing a combination of a steroid 5-alpha-reductase inhibitor and an alpha blocker, with said dosage form preferably having an extended shelf-life when compared to commercially available products containing the same active ingredients. The dosage form of the invention comprises: (A) a solid dispersion, comprising (i) an effective amount of a steroid 5-alpha-reductase inhibitor, preferably a hydrophobic steroid 5-alpha-reductase inhibitor and (ii) pharmaceutically acceptable non-active ingredients, and (B) pellets containing a modified release formulation which provides an effective amount of an alpha blocker and pharmaceutically acceptable non-active ingredients which provide a delayed release of said alpha blocker; wherein said hydrophobic steroid 5-alpha-reductase inhibitor is dutasteride and said alpha blocker is tamsulosin, and said components (A) and (B) are solid and are put together in a single dosage form.

The invention is also directed to a dosage form with a combination of a steroid 5-alpha-reductase inhibitor and an alpha blocker as previously disclosed for use in the treatment or prophylaxis of an androgen mediated disease or condition, preferably benign prostatic hyperplasia (BPH).

A third embodiment of the present invention is the process for preparing a dosage form with a combination of a steroid 5-alpha-reductase inhibitor and an alpha blocker as previously disclosed , preferably showing good bioavailability and increased patient compliance, said process comprising the following steps :
(i) dissolving the 5-alpha-reductase inhibitor with the hydrophilic polymer and the surfactant in a suitable solvent,
(ii) spraying the solution of step (i) on an inert pharmaceutical diluent carrier,
(iii) granulating the mixture of step (ii),
(iv) drying the mixture of step (iii),
(v) separately preparing modified release pellets containing an alpha blocker,
(vi) placing the granulate of (iii) and the pellets of (v) together in a single dosage form selected from capsules, tablets or sachets.

In preferred embodiments of the invention, the steroid 5-alpha-reductase inhibitor used in the dosage form and in the process of the invention is dutasteride and the alpha blocker is tamsulosin hydrochloride (i.e. tamsulosin in the form of hydrochloride salt).

In the most preferred embodiment, the dutasteride is present as a solid dispersion and the tamsulosin hydrochloride as modified release pellets in a hard gelatin capsule.

Finally, the invention is also directed to a method for the treatment or prophylaxis of an androgen mediated disease or condition comprising the administration to a mammal of an effective amount of dosage form as previously disclosed. In particular, this method is for the treatment or prophylaxis of benign prostatic hyperplasia.

### Brief Description of the Figures

Figure 1 illustrates a comparison of the Drug Release Profile between a commercially available product (Duodart® (◆)) and the composition of the present invention (○).
Figure 2 shows two dosage forms of dutasteride plus tamsulosin for comparison: (a) the commercially available product (Duodart®) and (b) the dosage form of the present invention.

### Description of the Invention

The present invention provides pharmaceutical compositions of tamsulosin and dutasteride for administration once a day wherein said composition provides better patient treatment with good bioavailability and good compliance.

In particular, the present invention relates to formulations combining dutasteride, in the form of a solid dispersion, and tamsulosin, in the form of modified release granules.

Dutasteride is completely insoluble in water, and in order to have acceptable bioavailability, commercial formulations (Avodart®) use biologically compatible solvents to dissolve the active ingredient. Avodart® is a soft gelatin capsule for oral administration containing 0.5 mg of dutasteride dissolved in a mixture of mono-diglycerides of caprylic-capric acid (such as that provided by Capmul™ MCM) and butylated hydroxytoluene. In this case, the active ingredient is in the solution and the solution is encapsulated in a soft gelatin capsule. But, soft gelatin capsules containing liquids usually have limited stability and a relatively short shelf-life period. In particular, there are several drawbacks, such as the risk of cross-linking of the gelatin over time, with the impact of rupturing of the capsule and reduced rate of drug release. Furthermore, the production of soft-capsule medications requires specialized equipment and a time-consuming production process.

Furthermore, the use of a dutasteride formulation in a soft gelatin capsule in liquid form makes the combination with tamsulosin unpractical, both from the standpoint of the patient as well as the industrial point of view. In fact, the insertion of a soft gelatin capsule of dutasteride within a hard gelatin capsule (or HPMC) containing tamsulosin, as in the case of the commercial product Duodart®, requires the need for a large capsule (size double zero) and a very complicated and time consuming production process.

The main problem in the oral delivery of dutasteride in solid dosage form is related to dissolution and absorption. Among the various techniques known in the art to increase the dissolution and absorption of the insoluble drug in the present invention, we selected solid dispersions of dutasteride.

We prepared solid dispersions with non-active ingredients comprising at least one ingredient selected from the group consisting of hydrophilic excipients, surfactants, inert carrier, and colorants. Preferred hydrophilic excipients include: polyethylene glycol, poloxamers, lactose, HPMC, HPC and polyvinylpyrrolidone. Particularly, the hydrophilic excipient polyvinylpyrrolidone (povidone, PVP K30®) is used in the solid dispersion of dutasteride in the invention.

Preferably, the solid dispersion of dutasteride contains a surfactant selected from Tween, Span, Pluronics, polyoxyethylene sorbitol esters or monoglycerides. The most preferred surfactant is polyoxyethylene sorbitol esters, in particular polyoxyethylene sorbitan monooleate (for example, Polysorbate 80, Tween 80®).

The solid dispersion of dutasteride of the invention is prepared by solubilizing the active ingredient and excipients in a suitable solvent. According to the invention, the preferred solvents are ethanol, a mixture of dichloromethane and ethanol, and isopropyl alcohol, which are used to solubilize dutasteride and the hydrophilic excipients. Of these, isopropyl alcohol is the most preferred.

The compositions were prepared by dissolving dutasteride and excipients in a proportion ranging from 0.5 to 15. Preferably, according to the invention, the solid dispersion of dutasteride is prepared from about 1:10 IPA / Ratio of excipients in isopropyl alcohol (15% solids). Briefly, the solution of povidone + dutasteride + Polysorbate 80 in isopropyl alcohol was sprayed on cornstarch, which acts as an inert carrier. After drying and removal of the solvent, the solid dispersion of dutasteride containing the granulate was calibrated and was ready to be introduced into the gelatin capsule. Other alternative inert carriers are lactose, microcrystalline cellulose, calcium phosphate, etc.

In a further embodiment of the present invention, oral dosage forms are prepared by adding dutasteride in a granular solid dispersion as described above, and tamsulosin hydrochloride filling a capsule, tablet or sachet. Preferably, the tamsulosin hydrochloride is introduced in a hard gelatin capsule.

Tamsulosin hydrochloride is present in the composition in a modified release formulation, in particular in a pellet form comprising a modified release formulation. The modified release formulation provides a prolonged release of the drug with appropriate plasma levels sustained over time so that a once a day formulation is feasible and some of the side effects associated with immediate release, such as hypotension, are avoided.

The preferred formulations of tamsulosin are modified release pellets, more preferably modified release enteric coated pellets.

Preferably, the enteric coating layer of the pellets of tamsulosin is a polymethacrylic acid derivative, or a cellulose derivative such as Eudragit L30D or cellulose acetate phthalate. More preferably, said enteric coating layer ranges from 5 to 20% w/w of the final granule weight.

Dry mixtures of acrylic enteric polymers that require an aqueous mixture are also suitable and available, such as, for example, Acryl-EZE ® from Colorcon.

Modified release excipients that may be used to control the dissolution of tamsulosin hydrochloride are derived from methacrylate, such as Eudragit 40D, Eudragit RL and Eudragit RS and mixtures thereof: ethylcellulose, such as Surelease® from Colorcon or Aquacoat from FMC Biopolymer.

The coating may be applied as an aqueous dispersion or as a solution in organic solvents. The amount of coating comprises 2 to 20% of the total weight of the composition. The coating may further include one or more components such as pore formers, plasticizers, wetting agents, etc. Examples of pore formers are hydroxypropylmethyl cellulose (HPMC), for example, METHOCEL®, polyethylene glycol, polyvinylpyrrolidone, etc.

Suitable plasticizers are triethyl citrate, dibutyl sebacate, triacetin, etc. The plasticizer is present in an amount of 1 to 40% by weight, preferably 5-10% of the total weight of the coating.

Modified release pellets of tamsulosin are commercially available from many sources or can be prepared using known techniques, using excipients and methodologies published in Remington "The Science and Practice of Pharmacy," 21st edition, 2005, and in the "Handbook of Pharmaceutical Excipients" 2012, 7th edition.

Both components (A) containing dutasteride - preferably in a solid dispersion - and (B) tamsulosin - preferably in pellets -, are approximately spherical particles, preferably introduced into a hard gelatin capsule. Also, preferably, the components (A) and (B) have different colors, for example, (A) is blue and (B) is red. The colorants used in the components (A) and (B) are those which are pharmaceutically acceptable and known in the art. One who is skilled in the art will know how to select the appropriate dye substance that must be compatible with the active and non-active ingredients of components (A) and (B). Surprisingly, in the preferred embodiment of the invention, this novel formulation not only has better patient compliance due to its lower dosage form, resulting from an easier and cheaper production process, but also provides a good stability and dissolution profile compared to the reference product Duodart®.

The components (A) and (B) are placed together in a single dosage form, which can be a capsule, tablet or sachet. Preferably, according to the invention, said unit dosage form is a capsule which may be made of gelatin, natural polysaccharides (such as carrageenan, pectin, curdlan, furcellaran and gellan gum), hydroxypropyl methylcellulose (HPMC) or similar. More preferably, according to the invention, the single dosage form is a hard gelatin capsule.

The preferred compositions of the invention are administered at a dose ranging from 0.1 to 10.0 mg/day of dutasteride and between 0.1 and 10.0 mg of tamsulosin. A common dosage and administration of the combination is about 0.5 mg once a day for each of the two drugs in a size zero capsule. Clearly, there is an advantage of a smaller dosage form, as in the present invention, in terms of improved compliance, especially for products such as this which are directed to older people who often have trouble swallowing medicines. For example, the size of the capsule of Duodart® is "00" (i.e., a capsule of 0.95 ml), whereas the capsule size of the dosage form of the present invention is "0" (i.e., a capsule of 0.68 ml) or smaller. This is a very important in the prevention of problems from ingestion of medications that may arise from the adherent characteristics of gelatin.

The dutasteride-tamsulosin compositions of the present invention may be administered in combination with other drugs, such as phosphodiesterase and antimuscarinic inhibitors (PDE5) for the treatment of BPH. The preferred antimuscarinic drug is tolterodine and the preferred PDE5 inhibitor is tadalafil.

The present invention will now be illustrated by the following examples. It is understood, however, that these examples are provided for the purpose of illustration only and that the invention is not intended to be limited by the examples. Formulations based on the system used in the examples can be formed by any suitable method known in the art.

### Example 1. Preparation of the composition of dutasteride

### Composition of dutasteride granules

| | |
|---|---|
| Dutasteride | 0.375% |
| Povidone K30 | 4.0% |
| Tween | 0.25% |
| Cornstarch | 95.375% |
| Isopropanol (removed during the process) | 25% |
| compared to solids | |

### Preparation Process

A solution of dutasteride, Povidone K30, Tween 80, and isopropanol was prepared in a container. The solution was added to the cornstarch in a high shear mixer and the mixture was granulated.

The wet granulate was passed through a square sieve of 4.75 millimeters with a cone mill machine, and then the granulated material was dried in a static oven at 45° C until reaching an LOD of 6%.

After drying, the dried granulate was passed through a sieve of 1.0 mm with the cone mill machine.

### Example 2. Preparation of tamulosin pellets

| *Composition of tamsulosin pellets* | |
|---|---|
| Tamsulosin Hydrochloride | 0.40 mg |
| Microcrystalline cellulose | 164.60 mg |
| Copolymer of methacrylic acid-ethyl acrylate (Eudragit L-30 D555) | 20.00 mg |
| Talc | 13.00 mg |
| Triethyl citrate | 2.00 mg |
| Purified Water | - |

### Production Process

Tamsulosin hydrochloride was mixed in a high shear mixer with talc (50% of total amount) and microcrystalline cellulose until a homogeneous powder mixture was obtained.

A suspension of Eudragit L30 D-55 (40% of total amount), triethyl citrate (40% of total amount) and water was prepared in a separate container.

The suspension was added to the powder mixture and the mixture was granulated, extruded and spheronized.

The resulting pellets were dried in a fluid bed dryer until the loss on drying (LOD) reached a value of between 2-4%.

The coating suspension was prepared by mixing triethyl citrate (60% of total amount), water, Eudragit L30 D-55 (60% of total amount) and talc (50% of total amount). The pellets were placed in a fluid bed coater and coated, until the coating was increased to about 10% of the mass of the pellet core.

### EXAMPLE 3. Preparation of tamsulosin pellets

| | |
|---|---|
| Tamsulosin Hydrochloride | 0.40 mg |
| Hydroxypropylcellulose (Klucel EF) | 0.20 mg |
| Ethylcellulose (Ethyl Cellulose NF) | 3.00 mg |
| Sodium lauryl sulfate | 2.00 mg |
| Sugar spheres NF 30/35 | 194.40 mg |
| Purified water | 30.00 mg |
| Ethanol | 120.00 mg |
| Hypromellose phthalate (HP-55) | 10.00 mg |
| Triethyl citrate | 2.00 mg |
| Talc | 10.0 mg |
| Purified water | 50.00 mg |
| Ethanol | 200.00 mg |

Tamsulosin HCl is dissolved in a mixture of ethyl alcohol and purified water using a mechanical stirrer until a clear solution is obtained. Hydroxypropyl cellulose (Klucel EF) is added to the solution, until it is completely dissolved.

Ethyl cellulose is added to the solution and mixing is continued until completely dissolved.

Then, the sodium lauryl sulfate is added to the solution and mixing is continued until completely dissolved. Once the drug is added, the suspension must be continuously stirred until the pulverization process is completed in order to prevent sedimentation.

Sugar spheres NF 30/35 are placed in a fluidized bed coater, and the drug suspension prepared above is sprayed onto the sugar beads.

Once the drug suspension has been consumed, the pellets are dried until the loss on drying (LOD) reaches an amount less than 2%.

Finally, the pellets are sieved through mesh screens of 20 and 50 mesh, and the pellets between 20 and 50 mesh are collected.

An enteric coating is then prepared. Triethyl citrate, dissolved in a mixture of purified water and ethyl alcohol, is prepared and stirred using a mechanical stirrer until the solution becomes clear. Hypromellose phthalate (HP-55) is added to the solution and stirred until completely dissolved.

The solution is then applied to the pellets in layers of active drugs prepared as described above, using a fluidized bed coater. Talc may be added to prevent adhesion.

Finally, the pellets are sieved through screens of 20 and 50 mesh and the pellets between screens of 20 and 50 mesh are collected.

Tamsulosin hydrochloride pellets are also commercially available from different sources, such as, for example, from Rottendorf Pharma GmbH (Germany) and RA Chem Pharma Ltd. (India).

### Example 4 Preparation of the final capsules

The combination of dutasteride in solid dispersion granules and pellets of tamsulosin hydrochloride obtained as described above, was prepared by filling the two components in size "0" hard gelatin capsules.

The process was conducted in an automatic bench top capsule filling machine; the In-Cap capsule filling machine (dott. Bonapace & C, Milan, Italy) equipped with two dosing stations:
1) First station: pellet filling station
2) Second station: powder dosing group/granulated with needles

The capsule filling machine was set to produce about 2,500 cps/h.

The final weight of the capsule was 430 mg.

### Example 5. In vitro release

To assess drug release, a dissolution test was administered by the following method:
USP apparatus II (paddle), with drains; 75 rpm;
Medium: acid phase: 0.1 N HCl (120 min), with 1% sodium lauryl sulfate, Buffer stage: buffer solution pH 6.8 (8 h);
Volume 750 ml (acid), 1000 ml (buffer);
Sampling time: 15, 30, 45, 60, 120 minutes (acid), 1, 2, 4, 6 and 8 hours (buffer).

**Table 1: Dissolution test performed on hard gelatin capsules of formulation 1 vs. the reference Duodart ®.**

| | Dutasteride | | Tamsulosin HCl | |
|---|---|---|---|---|
| Sampling Time (min) | Formulation Example 1 (n=6) | Duodart® (n=6) | Formulation Example 1 (n=6) | Duodart® (n=6) |
| 0 (*acid phase*) | 0 | 0 | 0 | 0 |
| 15 ( " ) | 98 | 50 | 0 | 0 |
| 30 ( " ) | 100 | 68 | 0 | 0 |
| 45 ( " ) | 100 | 84 | 0 | 0 |
| 60 ( " ) | 100 | 96 | 0 | 0 |
| 120 ( " ) | - | - | 5 | 0 |
| 120 + 0.5 hr (*buffer phase*) | - | - | 45 | 50 |
| 120 + 2 hr ( " ) | - | - | 70 | 75 |
| 120 + 4 hr ( " ) | - | - | 88 | 93 |
| 120 + 6 hr ( " ) | - | - | 98 | 100 |

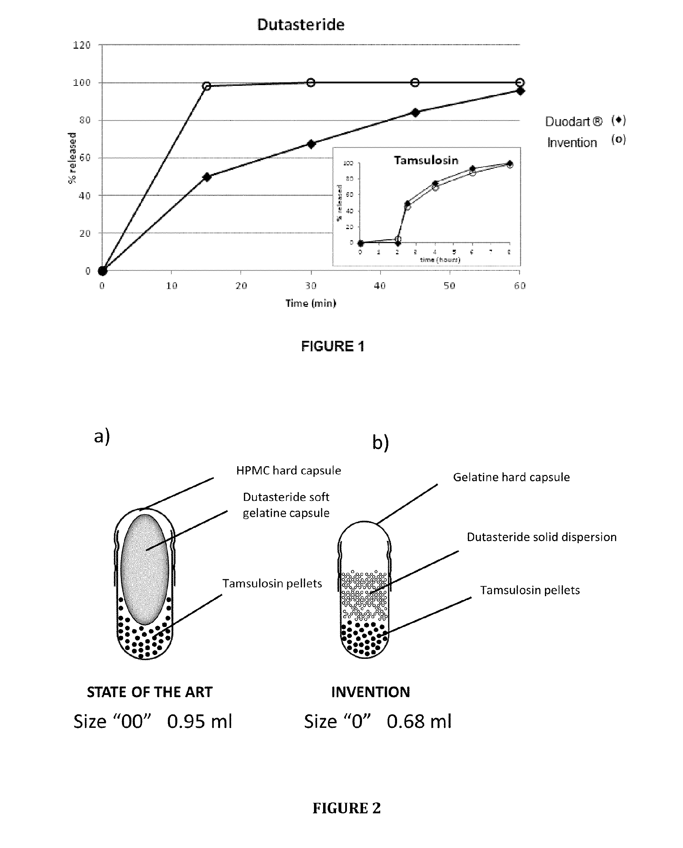

As illustrated in Figure 1, dutasteride in the solid dispersion contained in the dosage form of the invention, that is, hard gelatin capsules, showed a faster dissolution profile than the solution of Duodart®. In the lower right corner, the dissolution profile of tamsulosin pellets is shown. The test was performed in HCl 0.1 N for 2 hours and then in a buffer of 6.8 pH for 6 hours. The profiles are perfectly overlapping.

From Figure 1, it is possible to observe that the dissolution profile of dutasteride of the present invention is faster in the initial data points than the reference product, indicating an improvement in the release of dutasteride from solid dispersion. As expected, the release of tamsulosin is similar for the two formulations.

Figure 2 shows the difference between the dosage form of the invention and the dosage form of a commercially available product (Duodart®). The product known in the art comprises two capsules, the first of which is a hard capsule containing granules of modified release tamsulosin and a second capsule (within the first), which is a soft gelatin capsule containing a liquid comprising dutasteride. The dosage form of the invention contains both active ingredients (tamsulosin and dutasteride) in solid form, with dutasteride in a solid dispersion, and tamsulosin as a modified release pellet. As illustrated in Figure 2, the size of the reference product (prior art) is "00," whereas the size of the single dosage form (as a capsule) is "0."

## Claims

1. Dosage form comprising a steroid 5-alpha-reductase inhibitor and an alpha blocker, said dosage form comprising:
(A) a solid dispersion comprising:
(i) an effective amount of a steroid 5-alpha-reductase inhibitor
(ii) non-active pharmaceutically acceptable ingredients, and
(B) pellets containing a modified release formulation which provides an effective amount of an alpha blocker and non-active pharmaceutically acceptable ingredients which provide a delayed release of said alpha blocker;
wherein said hydrophobic steroid 5-alpha-reductase inhibitor is dutasteride and said alpha blocker is tamsulosin,
and said components (A) and (B) are solid and put together in a single dosage form.

2. The dosage form of claim 1, wherein the tamsulosin is in the form of hydrochloride salt.

3. The dosage form of claim 1, wherein said single dosage form is selected from capsule, tablet or sachet.

4. The dosage form of claim 3, wherein said unit dosage form is a capsule made natural polysaccharides of gelatin, HPMC or similar.

5. The dosage form of claim 4, wherein said unit dosage form is a hard gelatin capsule.

6. The dosage form of claim 1, wherein said non-active ingredients comprise at least one ingredient selected from the group consisting of hydrophilic excipients, surfactants, inert carrier, and colorants.

7. The dosage form of claim 6, wherein said hydrophilic excipients include: polyethylene glycol, poloxamers, lactose, HPMC, HPC, lactose, polyvinylpyrrolidone.

8. The dosage form of claim 7, wherein said hydrophilic excipient is polyvinylpyrrolidone.

9. The dosage form of claim 6, wherein said surfactant is selected from Tween, Span, Pluronics, polyoxyethylene sorbitol esters or monoglycerides.

10. The dosage form of claim 9, wherein said surfactant is a polyoxyethylene sorbitol ester.

11. The dosage form of claim 1, wherein tamsulosin is into a pellet form comprising a modified release formulation.

12. The dosage form of claim 1, wherein both components (A) and (B) are approximately spherical particles introduced into a hard gelatin capsule.

13. The process for preparing a dosage form containing a steroid 5-alpha-reductase inhibitor and an alpha blocker, as defined in claims 1-12, said process comprising the steps of:
(i) dissolving the 5-alpha-reductase inhibitor with the hydrophilic polymer and the surfactant, in a suitable solvent,
(ii) spraying the solution of step (i) on an inert pharmaceutical carrier
(iii) granulating the mixture of step (ii),
(iv) drying the mixture of step (iii)
(v) separately preparing modified release pellets containing an alpha blocker,
(vi) placing granulate of (iii) and pellets of (v) together into a single dosage form selected from capsule, tablet or sachet.

14. The process of claim 13, wherein the unit dosage form is a capsule and step (vi) is the filling of hard capsules with the dried granulate (iv) and the alpha blocker modified-release pellets (v).

15. The process of claim 13, wherein said steroid 5-alpha-reductase inhibitor is dutasteride.

16. The process of claim 13, wherein the said alpha blocker is tamsulosin hydrochloride.

17. Dosage form as defined in any of claims 1-12, for use in the treatment or prophylaxis of an androgen mediated disease or condition.

18. Dosage form according to claim 17, for use in the treatment or prophylaxis of benign prostatic hyperplasia.
